Europäisches Patentamt

European Patent Office

Office européen des brevets

(19)

(11) Publication number: **0 353 689**
**A2**

## (12) EUROPEAN PATENT APPLICATION

(21) Application number: **89114108.7**

(22) Date of filing: **31.07.89**

(51) Int. Cl.⁴: **A01N 63/00 , C12N 1/20 ,**
**//(C12N1/20,C12R1:01,1:38,**
**1:39,1:40)**

The microorganism(s) has (have) been deposited with CNCM under numbers I 805 + I 806.

(30) Priority: **02.08.88 IL 87322**

(43) Date of publication of application:
**07.02.90 Bulletin 90/06**

(84) Designated Contracting States:
**AT BE CH DE ES FR GB GR IT LI LU NL SE**

(71) Applicant: **RAMOT UNIVERSITY AUTHORITY FOR APPLIED RESEARCH & INDUSTRIAL DEVELOPMENT LTD.**
**32, Hauniversita Street Ramat-Aviv**
**Tel-Aviv 69975(IL)**

(72) Inventor: **Eyal, Zahir**
**7 Akelton Street**
**Hod Hasharon 45 207(IL)**
Inventor: **Chet, Ilan**
**Shikun Ezrahi**
**Nes-Ziona 70 400(IL)**
Inventor: **Fleishman, Moshe**
**7 Pichman Street Ramat-Aviv**
**Tel-Aviv 69 207(IL)**
Inventor: **Levy, Edna**
**18 Tel-Hai Street**
**Kfar-Saba 44 456(IL)**

(74) Representative: **Patentanwälte Grünecker, Kinkeldey, Stockmair & Partner**
**Maximilianstrasse 58**
**D-8000 München 22(DE)**

(54) **Method of controlling foliar diseases caused by fungal pathogens with fungicidal bacteria and novel pure cultures of fungicidal bacteria.**

(57) Foliar fungal pathogens may be effectively controlled by fungicidal bacteria which naturally colonize the leaves together with these pathogens. These bacteria are isolated and then formulated into phytological fungicidal compositions which may then be applied to foliage for combating fungal infestations.

Several strains of such bacteria have been isolated into pure cultures and used effectively for controlling fungal infestations.

EP 0 353 689 A2

# METHOD OF CONTROLLING FOLIAR DISEASES CAUSED BY FUNGAL PATHOGENS WITH FUNGICIDAL BACTERIA AND NOVEL PURE CULTURES OF FUNGICIDAL BACTERIA

## FIELD OF THE INVENTION

The invention concerns methods and compositions for the bacterial control of plant leaf fungal pathogens, and novel pure cultures of fungicidal bacteria.

## BACKGROUND OF THE INVENTION

Due to the hazardous environmental effects of conventional chemical pesticides, there has been a growing interest in recent years in biopesticides, i.e. microorganisms capable of destroying or inhibiting pests. Such a control has the advantage of being target specific and not to pollute the environment.

Fungi such as *Septoria tritici* and *Puccinia recondita*, are known to infect foliage of cereal plants and to reduce the yield of crops, the former fungi being the causative organism of the plant leaf disease known as *Septoria tritici* blotch of wheat and the latter of wheat leaf rust. The need for an effective control of such fungal diseases by biofungicides has been long felt, but to date no effective biological control of the causal pathogens has been found.

The use of microorganisms of the species *Bacillus subtilis* for controlling leaf rust in bean plants has been disclosed in U.S. Patent 4582704. In accordance with that patent, an amount of a late stationary phase culture of *B. subtilis* is released onto leaves of infested plants. The patent also discloses the use of the supernatant of the culture medium of the control of leaf-rust achieved in accordance with that the disclosure in patent is not satisfactory.

It is the object of the present invention to provide effective biological means for controlling foliar fungal diseases in plants.

## SUMMARY OF THE INVENTION

The present invention is based on the realization that an effective control of fungal pathogens causing foliar diseases may be achieved by bacteria which are fungal antagonist and which naturally colonize the leaves together with the target pathogens. Such bacteria are capable of destroying these foliar pathogens and inhibit their proliferation, in a target specific manner and without having any hazardous effect on the environment. It has, moreover, surprisingly been found in accordance with the invention that a growth medium in which the antagonistic bacteria were cultured and from which they were recovered, a supernatant or an extract thereof as well as solid products obtainable from such supernatant or extract are also effective in the destruction of foliar pathogens. Such supernatants, extracts and solid products will be referred to hereinafter collectively as "residual medium" and "residual media".

Thus, in accordance with the present invention there is provided a method of controlling fungal pathogens causing foliar diseases in plants, comprising applying onto a plant's foliage an effective amount of bacteria that are antagonists to fungal pathogens and which naturally colonize plants' foliage together therewith (hereinafter referred to as "fungicidal bacteria"), and/or an effective amount of a residual medium as herein defined.

The invention also provides a phytological liquid fungicidal composition comprising an effective amount of fungicidal bacteria and/or a residual medium, both as herein defined together with a phytologically acceptable liquid carrier. In accordance with one embodiment the liquid fungicidal composition is aqueous.

The invention further provides pure cultures of fungicidal bacteria, characterized in that they are isolated from leaves of plants infected with fungal pathogens causing foliar disease.

In accordance with the present invention, bacterial strains of the genera *Pseudomonas* and *Agrobacterium* have been isolated for the first time in pure culture form from leaves of wheat plants infected with fungi, and it was found that such bacteria have the ability of suppressing and inhibiting the proliferation of certain fungal wheat pathogens *in vitro* and on wheat seedlings.

Thus the present invention further provides a pure culture of a fungicidal bacterium selected from the genera *Pseudomonas* and *Agrobacterium*.

The present invention also provides a method for the preparation of a pure culture of fungicidal bacteria as defined herein, comprising preparing a growth mixture from a leaf infested with foliar fungi and a bacterial growth medium, incubating such growth mixture until bacteria have proliferated and then subjecting a sample of the growth mixture to purification operations as known per se.

Several strains belonging to the genera *Pseudomonas* and *Agrobacterium* have been isolated. Some of these were identified as belonging to the species *P. putida* (e.g. one that was termed "BK86-6/1"), others to the species *A. radiobacter* (e.g. one which was termed "NO86-2/3") and yet others were identified as belonging to the species *P. fluorescence*.

The fungicidal bacteria are capable of inhibiting and destroying the leaf fungus by either one or a combination of the following mechanisms:

a) Secretion of anti-fungal vapours;

b) Secretion of antibiotic compounds;

c) Competition for nutrients on the leaf surface, which is usually poor in nutrients.

Various experiments performed in accordance with the present invention, suggest that each one of these three mechanisms plays a role in the suppression of the target fungi, and probably there is an additive combination of all three.

A pure culture according to the present invention may be mutated in a controlled manner by subjecting it to the action of various mutagens, such as radioactive substances, chemical mutagens and - or X-irradiation, in order to obtain a new mutant strain which has an improved fungicidal activity or other improved properties over the parent strain, e.g. longer viability, faster growth rate and the like. The terms "bacteria", "fungicidal bacteria" and the like used herein apply to both a parent fungicidal strain as first obtained in pure culture form, and a fungicidal active mutant thereof.

Upon isolation, the bacteria can be conserved for short periods of time, i.e. weeks to months, in a living culture, but care must then be taken that from time to time fresh medium is added and waste products and excess bacteria are removed, or that a sample of the culture is transferred to a new medium. For a longer term, the culture should preferably be frozen.

The preparation of commercial compositions of fungicidal bacteria requires large amounts of such bacteria, and those are obtainable by fermentation.

## STATEMENT OF DEPOSIT

Several bacterial strains have been isolated in accordance with the present invention. Among these, two were deposited with the culture collection at the Institut Pasteur, (Collection Nationale de Cultures de Microorganismes - CNCM) and the details of these deposited strains are as follows.

TABLE 1

| Genus | Species | Strain | Accession Number | Deposition Date |
|---|---|---|---|---|
| *Pseudomonas* | *P.putida* | BK86-6/1 | I-806 | October 10, 1988 |
| *Agrobacterium* | *A. radiobacter* | NO86-2/3 | I-805 | October 10, 1988 |

## DETAILED DESCRIPTION OF THE INVENTION

In the following description reference will at times be made to the species *P. Putida* to which the strain "BK86-6/1" belongs and to the species *A. radiobacter* to which strain "NO86-2/3" belongs. It is obvious to the man of the art that this description applies equally well to all other species and strains within the scope of the present invention in general and to those belonging to the genera *Pseudomonas* and *Agrobacteria* in particular.

## I. Culture purification

The fungicidal bacteria are obtained from the surface of leaves infested with fungus, such as, for example, from leaves of cereal plants infected with *Septoria tritici* or *Puccinia recondita*. The procedure is generally as follows:

Leaves infested with fungus are withdrawn, washed and thereafter may be extracted with a mineral solution. The crushed leaves of this extract are then transferred to a growth medium suitable for growth of bacteria such as King's medium B (KMB). After a sufficient incubation time, e.g. 24 hrs a sample of the medium, may be diluted and if desired transferred to a culturing plate comprising an agar matrix, which agar is prepared with a suitable growth medium such as King's medium B. Single colonies may then be suspended in a medium and after diluting the bacterial suspension, a sample may again be transferred to an additional culture plate having a similar agar matrix. Resuspending, diluting and reseeding may be carried out several times if desired. The so obtained pure culture have to be screened for fungicidal activity. Such a screening is first performed *in vitro*, for example by an inhibition ring test *tritici*. Later *in situ* tests may follow.

## II. Storage of the microorganisms

Storage of *P. putida* or *A. radiobacter* may either be short term or long term. For short term storage the cultures may be conserved by growing them in a growth medium, e.g. agar plates with a bacterial growth medium. On such a growth medium *P. putida* produces fluorescent pigments. In order to keep the culture viable, a sample thereof should periodically (e.g. once every two weeks) be transferred into a new medium, or a new medium should periodically be added to the culturing medium and access of bacteria and waste products should then be removed.

For long term storage, *P. putida* or *A. radiobacter* can be frozen and the procedure of freezing may, for example, be as follows: the bacterial culture is grown in a liquid King's medium B (KMB) until logarithmic growth phase is reached. The bacterial suspension is then mixed with equal volumes of a freezing medium consisting of (g/l) $K_2HPO_4$-12.6, $KH_2PO_4$-3.6, Na-citrate-0.9, $MgSO_4.7H_2$-0.18, $(NH_4)_2SO_4$-1.8, Glycerol-88, and then left at 25°C for about 30 min. prior to storage and thereafter the suspension may be frozen to -70°C. The addition of a freezing medium is necessary in order to recover a good proportion of viable cells after thawing the stored culture.

Long term storage can also be achieved by freeze drying in accordance with methods known per se. Briefly, such freeze drying may, for example, be as follows: bacterial cultures are grown in a liquid medium as above until logarithmic growth phase is reached. The bacterial suspension is then centrifuged, e.g. at 12,000 g for 10 min. The resulting bacterial pellet obtained after removing the supernatant is resuspended in a sterile 10% skimmed milk powder solution, followed by thorough mixing. Thereafter, the samples are frozen in dry ice/acetone and are then lyophilized. The freeze dried bacteria may thus be stored over prolonged periods at room temperature.

By such storage the original characteristics and traits of the pure cultures are present.

## III. Fermentation.

*P. putida* and *A. radiobacter* were found to grow very well on many industrial waste products. Thus, for example, very good growth results were achieved with a medium containing 2g/l Sheftones (a cheap peptone from Sheffield Products Ltd., Norwich NY) or 2g/l cottonseed meal (Pharmamedia, Traders Proteins, Fort Worth, Texas). These media can be supplemented, for example, with 0.1% corn-steep liquor which is a by-product of the starch industry. In addition, many other media containing a variety of cheap industrial waste products can be used for the fermentation.

The fermentation in the above media was found to yield $10^{10}$-$10^{11}$ bacterial cells/ml within about twenty four hours of fermentation. The bacteria grown in these fermentation media preserve their original characteristics which is evident by the restoration of fluorescence when grown on a King's B agar and the preservation of their ability to inhibit the *in vitro* growth of fungus such as *Septoria tritici* which under

4

normal conditions grows nicely on solid (e.g. agar) King's medium B. Furthermore, and of primary importance, *P. putida* and *A. radiobacter* preserve their ability to inhibit *in situ*, symptoms of various fungal diseases such as *Puccinia recondita* (leaf rust) and *Septoria tritici* (*Septoria tritici* leaf blotch).

IV. Formulation.

The *P. putida* is formulated into a liquid composition and as such applied to plant's foliage.

Due to the waxy surface of leaves, liquid tends to form drops when sprayed thereon, and consequently the sprayed composition will be unequally distributed over the leaves' surface. In order to ensure uniform distribution over the leaf's surface, compositions according to the invention preferably also contain effective amounts of a suitable surfactant such as, for example, various thinners e.g. Biofilm (trade mark, Colloidal Products, U.S.A.), which may be incorporated at a concentration of about 0.2%.

## DESCRIPTION OF SOME SPECIFIC EMBODIMENTS

In the following, some examples of specific embodiments are described, it being understood that these examples are meant only to illustrate the present invention and therefore they should not be considered as limiting. It is obvious to the man skilled in the art that the various modifications within the scope of the invention as defined in the claims are possible.

EXAMPLE 1

Purification of bacterial culture

Wheat leaves infested with *S. tritici* were cut into pieces and then the cut pieces were processed in one of the following ways:

(i) Cut pieces of the leaves were placed on either Nutrient Agar or KMB plates. After 24 hrs of incubation, bacterial colomers were isolated and subjected to further screening by determining their ability to inhibit development of *S. tritici in vitro* (see Example 4 below).

(ii) Cut pieces of the leaves were placed in a liquid mineral solution. After shaking for 5 hrs serial dilutions were made which were then plated on Nutrient Agar or KMB plates. Single bacterial colonies were then isolated and screened as above.

EXAMPLE 2

Growth medium

The isolated bacteria were grown on agar plates, the agar having been prepared with King's B medium which consisted of (in g/l unless otherwise stated), Proteose peptone-20, glycerol-10ml, aspargine-2.5, $K_2HPO_4$-1.5, $MgSO_4.7H_2O$-1.5 and agar-20.

The optimal pH was found to be about 6.0-8.2. The optimal temperature was found to be about 30° C.

On this medium BK86-6/1 produces fluorescent pigments, which is a good indicator of the cells' viability.

In order to preserve the culture's viability, once every two weeks a sample thereof was suspended in a liquid and grown onto a new agar plate containing the same medium.

Other media such as the Lurie Broth (with and without glucose) and Tryptic Soy Broth (TSB) were also found to be suitable.

EXAMPLE 3

Fermentation

Strains BK86-6/1 and NO86-2/3 were grown in media containing either "Sheftones") or cotton seed meal (Pharmamedica, Traders Proteins), both at a concentration of 2g/l were tested. In each case 0.1% corn steep liquor (which is a by-product of the starch industry) was added.

Cell yield in each case was found to be $10^{10}$-$10^{11}$ cells/ml after about 24 hrs of fermentation. The bacteria retained their properties following fermentation, which was evident in the particular case of strain BK86-6/1 by fluorescence when grown on King's B medium, their ability of all of them to inhibit growth of *S. tritici* on agar, and their ability to inhibit *in vivo* the disease symptoms of leaf rust and *Septoria tritici* blotch.

EXAMPLE 4

*In vitro* inhibition of development of *Septoria tritici*

The ability of the various isolated fungicidal bacterial strains to inhibit the *in vitro* development of *Septoria tritici* was tested. Three different tests, the results of which are shown in Table 2 below, were made as follows:

(i) Inhibition on King's medium B: Bacteria were placed on KMB on one pole of the plate. Three days later, conidia of *S. tritici* were placed on the opposite corner of the plate. The ability of the tested bacteria to prevent the growth of *S. tritici* is indicated as " + " in Table 2.

(ii) TLC (thin layer chromatography) *Septoria* bioassays: The test was a modification of the method of Howell & Stipanovic (1979). Bacteria were grown on KMB for 7 days, after which 10% acetone was added and the suspension sonicated for 45 min. and then shaken for 24 hrs. The suspension was then filtered through cheese-cloth and the filtrate was then centrifuged at 12,000g for 40 min. The supernatant was evaporated to remove the acetone and 5g of NaCl per 100 ml of aqueous fraction was added.

100 ml of dichloromethane was added to the aqueous solution. The organic phase was separated and evaporated to dryness. The crude extract obtained was applied on silica gel (60 $F_{254}$) TLC plates. The plates were developed in chloroform acetate (90:10 v/v) and dried. A spore suspension of *S. tritici* ($10^7$) spores/ml) was sprayed onto the plates. The melanin producing isolate developed a dark mycelial mat on the TLC plates except at the location where inhibitory substances were present. The diameter of the inhibition zone was measured and its results are presented in Table 2 below.

(iii) Paper disc diffusion agar bioassay: The crude extract obtained from the organic phase (see above) was dissolved in chloroform, dried and applied on sterile 0.5 min filter paper. The disc was placed on PDA (Potato Dextrose Agar) which was earlier suspended with spores of *S. tritici*. Paper discs impregnated in chloroform without spores were used as control. A " + " in the respective column in Table 2 indicates the presence of an inhibition zone around the discs.

The results of such a test are shown in the following Table 2:

TABLE 2

| Strain (*) | In vitro Inhibition of Septoria tritici | | |
|---|---|---|---|
| | King's B medium | TLC (diam) | discs |
| 2/3 | + | 5.0 | + |
| 6/1 | + | 1.6 | - |
| 3/3 | + | 2.5 | - |
| C | + | 2; 4.5 | + |
| E | + | 2.5 | ± |
| 5/2 | + | 0 | - |
| 5/4 | + | 1.8 | ± |
| 4/2 | + | 2.3 | ± |

(*) 2/3-CNCM, I-805; 6/1-CNCM, I-806; 3/3-a strain belonging to the species *P. fluorescence*; C, E, 5/2, 5/4 and 4/2-uncharacterized strains isolated in accordance with Example 1.

The above results show that the fungicidal bacterial strains inhibit the growth of **S. tritici** *in vitro*, and additionally that such inhibition is also exerted by the medium wherein such bacteria had been grown.

EXAMPLE 5:

Inhibition of fungal growth *in situ*.

Plants affected with either of **Pucinia recondita** (leaf rust) and **Septoria tritici** (leaf blotch) were sprayed with a composition containing between $10^{10}$ to $10^{11}$/cells/ml together with 3% maltose and 0.2% biofilm.

The ability of the various strains of fungicidal bacteria to suppress development of leaf rust **Puccinia recondita** on seedlings of the commercial wheat cultivars CEEON and SHAFIR, is shown in the following Table 3.

TABLE 3:

| | In situ suppression of pustule numbers/leaf area | | | |
|---|---|---|---|---|
| Strain (*) | CEEON (%) | Ranking | SHAFIR (1%) | Ranking |
| 2/3 | 95.8 | 1 | 97.1 | 1 |
| 6/1 | 79.7 | 2 | 85.0 | 2 |
| 3/3 | 77.7 | 3 | 84.1 | 3 |
| C | 70.7 | 4 | 78.5 | 5 |
| E | 56.1 | 6 | 77.1 | 6 |
| 5/2 | 51.9 | 7 | 64.9 | 7 |
| 5/4 | 46.3 | 8 | 59.5 | 8 |
| LECI | 70.0 | 5 | 83.9 | 4 |

(*) CNCM, I-805; 6/1-CNCM, I-806; 3/3 a strain of the species *P. fluorescence* isolated in accordance with Eample 1; C, E, 5/2 and 5/4 -uncharacterized strains isolated in accordance with Example 1.

EP 0 353 689 A2

The ability of different strains in various preparations to suppress symptoms caused by *Septoria tritici* on wheat seedlings is shown in the following Table 4:

TABLE 4:

| Strain (c) | (*) Percent(a) suppression of pycnidia coverage (b) | | |
|---|---|---|---|
| | Bacteria + medium (d) | Bacteria + H$_2$O (e) | Medium-bacteria (f) |
| C | 96.6 | 80.4 | 86.6 |
| E | 84.3 | 84.8 | 65.6 |
| 2/3 | 95.2 | 85.9 | 95.3 |
| 3/3B | 91.6 | 94.5 | 83.2 |
| 4/2 | 87.0 | 84.1 | 79.7 |
| 5/2 | 91.7 | 32.3 | 93.2 |
| 5/4 | 92.9 | 61.0 | 69.3 |
| 6/1 | 90.4 | 65.7 | 83.1 |

(a) The results are in % inhibition versus control.

(b) Conidia of *Septoria tritici* isolate ISR8036;

(c) 2/3 - CNCM,I-805; 6/1-CNCM,I-806; 3/3-an isolate belonging to the species *P. fluorescence* isolated in accordance with Example; C, E, 4/2, 5/2, 5/4 - uncharacterised strains of bacteria isolated in accordance with Eample 1.

(d) Isolate grown in liquid KMB and suspension sprayed onto wheat seedlings;

(e) Isolate grown in liquid KMB, centrifuged and bacteria resuspended in H$_2$O prior to spraying;

(f) Isolate grown in liquid KMB, centrifuged and supernatant sprayed onto seedlings.

These results demonstrate the ability of the fungicidal bacteria to inhibit the leaf's fungus suppression of symtoms can also be achieved by fractions derived from the medium where these bacteria were grown.

## EXAMPLE 6

*In situ* test of the antagonistic activity of some of the isolated strains under preliminary field tests

The field trial was conducted under commercial wheat growing conditions at Saad which is situated in the northern part of the Negev desert, Israel, in a plot where wheat was also grown in the previous season. The susceptible cultivar Shafir which was chosen for the trials is the most widely grown wheat cultivar in Israel. The plants were grown in 10m$^2$ microplots in four replications of each treatment. Each plot was separated by a 2.5m unsown buffer zone from all directions in order to eliminate inter-plot interference.

Each of the plots was subjected to one of the following treatments:

a) Six treatments with the isolated micro organisms of one of the following strains - 6/1, 3/3B, E, 2/3, 5/2 and C;

b) treatment with a commercial fungicide - "TILT" (propiconazole EC 250, (manufactured by Ciba Geigy, Basel, Switzerland).

c) untreated control.

The bacterial compositions included a bacterial suspension in nutrient broth at adjusted concentration of about 1x10$^7$Cfu/ml (colony forming unit). Each plot was sprayed with 150 ml suspension to which 0.5 ml of biofilm was added. Bacterial preparations were applied weekly with low pressure sprayers six times during the season, during evenings on dewy or rainy days. The application of the bacterial preparation was initiated when *Septoria tritici* plot reached pycnidia average of 5% on the flag leaf minus 2 (at growth stage 59) on March 18, 5% on the flag leaf minus 2 (at growth stage 59) on March 18, 1987 and continued at weekly intervals on March 26, April 4, April 8, April 16 and April 24, 1987.

8

TILT was applied in an amount of 125 grams (a.i.) per hectare per application in each of two applications on March 26, 1987 and twelve days afterwards, on April 16, 1987. This application rate and amount of applied fungicide is in accordance with accepted agricultural application rates.

The timing of bacterial preparation and fungicide application is shown in the following Table 5:

TABLE 5:

| Application date | Days from seedl. emergence | Growth stage | Description |
|---|---|---|---|
| March 18, 1987 | 81 | 59 | Emergence of ear complete |
| March 26, 1987* | 88 | 64 | Flowering halfway |
| April 2, 1987 | 96 | 68 | Flowering complete |
| April 8, 1987 | 102 | 70 | Kernel water ripe |
| April 16, 1987* | 109 | 73 | Early milk |
| April 23, 1987 | 117 | 77 | Late milk |

*TILT applications

In the tested plots pycnidia coverage of **Septoria tritici** and residual green leaf area was assessed three times during the season on the four uppermost leaves. The plants were then harvested after ripening for evaluation of the components.

The result of the blotch coverage and the residual green leaf are summarized in the following Table 6:

TABLE 6:

| Treatment | No. of Applicat. | Area under disease progress curve | % reduc. | Area under residual green leaf | % gain |
|---|---|---|---|---|---|
| 6/1 | 6 | 129.7 | 51.5 | 55.9 | 120.0 |
| 3/3B | 6 | 166.9 | 37.6 | 52.5 | 106.7 |
| E | 6 | 185.4 | 30.6 | 34.4 | 35.4 |
| 2/3 | 6 | 144.5 | 45.9 | 41.3 | 62.6 |
| 5/2 | 6 | 182.7 | 31.6 | 44.4 | 74.8 |
| C | 6 | 180.1 | 32.6 | 35.9 | 41.3 |
| TILT | 2 | 86.3 | 67.7 | 75.0 | 195.3 |
| CONTROL | 0 | 267.3 | -- | 25.4 | -- |

The above results show that strains "BK86-6/1" and "NO86-2/3" decreased the amount of disease by 51.5% and 45.9% respectively, as compared to a decrease in disease coverage of 67.7%, by TILT. It should be noted however that the difference between these three results is not statistically significant, while the difference between each of them and the control, is.

While all bacterial preparations showed a statistically significant difference from the control when measuring the area under disease progress curve, the difference between them and the control when measuring the residual green leaf was not as significant.

The effect of various treatments on the yield as expressed by kernel weight is shown in Table 7:

TABLE 7

| Treatment | 1000-kernel weight | % gain |
|-----------|--------------------|--------|
| 6/1 | 45.11 | 6.36 |
| 3/3 | 45.03 | 5.82 |
| E | 42.97 | 1.30 |
| 2/3 | 44.03 | 3.68 |
| 5/2 | 43.82 | 3.22 |
| C | 43.55 | 2.61 |
| Tilt | 46.45 | 8.69 |
| Control | 42.41 | -- |

It is evidenced by the above results that these cultures provided a significant reduction in disease severity which resulted in significant gains in kernel weights which is the yield component parameter most affected by foliar diseases.

The above results are rather unique and such an effective biocontrol of foliage disease of wheat has not been previously reported.

**Claims**

1. A method of controlling foliar fungal infestations in plants, comprising applying onto a plant's foliage an effective amount of a fungicidal agent selected from the group consisting of bacteria that are fungal antagonists and which naturally cohabitate plants' foliage with foliar fungi, fungicidally active mutants thereof and a residual medium as herein defined.

2. A method according to claim 1, wherein said bacteria are of the genus *Pseudomonas* or *Agrobacterium*.

3. A method according to claim 2, wherein said bacteria are of the species *P. putida*, *P. fluorescence* or *A. radiobacter*.

4. A method according to claim 3, wherein the bacteria are of the strain BK86-6/1 (CNCM,I-806), NO86-2/3 (CNCM,I-805), or fungicidally active mutants thereof.

5. A phytological liquid fungicidal composition comprising an effective amount of a fungicidal agent selected from the group consisting of fungicidal bacteria that are fungal antagonists and which naturally cohabitate plants' foliage with foliar fungi; fungicidally active mutants thereof and an effective amount of a residual medium as herein defined together with a phytologically acceptable liquid carrier.

6. A fungicidal composition according to Claim 5, wherein said liquid carrier is aqueous.

7. A composition according to Claim 5, wherein said bacteria are of the genus *Pseudomonas Agrobacterium*.

8. A composition according to Claim 7, wherein said bacteria are of the species **P. putida**, **P. fluorescence** or **A. radiobacter**.

9. A composition according to Claim 8, wherein said bacteria are of the strain BK86-6/1 (CNCM,I-806), (NO86-2/3,I-805) or fungicidally active mutants thereof.

10. A composition according to any one of Claims 5 to 9, containing also a surfactant.

11. A composition according to Claim 10, wherein said surfactant is Biofilm (trade mark).

12. A pure culture of fungicidal bacteria that are fungal antagonists and which naturally cohabitate plants' foliage with foliar fungi or are fungicidally active mutants thereof.

13. A pure culture of fungicidal bacteria according to Claim 12 being of the genus **Pseudomonas** or **Agrobacterium**.

14. A pure culture according to Claim 13, wherein the bacteria are of the species of **P. pudita**, **P. fluorescence** or **A. radiobacter**.

15. A pure culture according to Claim 14, wherein the bacteria are of the strain BK86-6/1 (CNCM, I-806), N806-2/3 (CNCM, I-805) or fungicidally active mutants thereof.

16. A method for the preparation of a pure culture of fungicidal bacteria that are antagonistic to foliar disease causing fungi and which naturally colonize plant's foliage together with such fungi, comprising preparing a growth mixture -from a leaf infested with foliar pathogenic fungi and a bacterial growth medium, incubating such growth mixture until bacteria have proliferated and then subjecting a sample of said growth mixture to purification operations as known per se.

17. A pure bacterial culture obtained by the method according to Claim 16.

18. A method according to Claim 16, subjecting the obtained pure culture to controlled mutagenic treatment as known per se.

19. A pure bacterial culture obtained by the method according to Claim 16.